# EUROPEAN PATENT APPLICATION

(11) **EP 3 061 499 A1**
(43) Date of publication of application: **31.08.2016**
(21) Application number: 15156730.2
(22) Date of filing: 26.02.2015
(51) Int. Cl.: A61Q 5/06, A61K 8/365, A61K 8/36, A45D 7/06

(54) **Method for providing visual effects on fibres**

(71) Applicant: The Procter & Gamble Company, Cincinnati, OH 45202 (US)
(72) Inventor: Sutton, Richard, Cincinnati, OH Ohio 45202 (US); Smith, Steven, Cincinnati, OH Ohio 45202 (US)
(74) Representative: Sauvaître, Thibault Bruno

(57) **Abstract**

Method and kit for providing visual effects on fibres, for example human keratin fibres. In the method, fibres are provided coated with a composition. The composition comprises monomer(s) and optionally a cosmetically acceptable carrier. The monomer(s) are capable of forming a polymer after exposure to heat. The monomer(s) are selected from the group consisting of acrylates monomers, methacrylate monomers, acrylamide monomers, methacrylamide monomers, styrene monomers, vinyl pyrolidinone monomers, vinylpyrrolidone monomers, and mixtures thereof. The monomer(s) have a molecular weight in the range from 100 g/mol to 5000 g/mol. The monomer(s) have a functionality of between 1 and 100. The composition has a kinematic viscosity of from 0.5 cSt to 1500 cSt, measured at 23°C. In the method, a forming means is applied onto the fibres. The forming means orders the composition into a pattern and is a conductor. In the method, the temperature of the forming means is raised to a temperature of from 40°C to 150°C for up to 15 minutes and the the forming means is removed from the fibres.

## Description

### FIELD OF THE INVENTION

Method and kit for providing visual effects on fibres, for example on human keratin fibres.

### BACKGROUND OF THE INVENTION

Methods for providing visual effects on hair are known in the art. Fibre coloration with little or no colorants occurs in nature. Peacock feathers, for example, are known to have little or no pigmentation. The striking colors in peacock feathers are produced primarily from diffraction of incident light from nanometer scale branches of the peacock feathers. Such an effect is desired for human hair.

US2013/0081646A1 discloses a hair treatment process comprising: coating hair with a fluid that includes a suitable polymer having a suitable glass transition temperature, inserting the hair coated with the fluid into a pressing device, the pressing device including a heating block having a configured with a nanopattern designed to diffract incident light into dispersed colors, the pressing device capable of transforming the fluid-coated hair into a hair-polymer film composite, the composite having a surface with a nanostructured surface pattern that is complementary to the surface pattern from the heating block, pressing the hair coated with the fluid under conditions suitable for forming the composite, cooling the composite and removing it from the pressing device, wherein polychromatic light incident on the nanostructured surface pattern is diffracted into dispersed colors that are visible on the nanostructured surface pattern of the composite.

WO2014/160904A1 discloses a hair treatment process for providing dispersed colors by light diffraction including (a) coating the hair with a material comprising a polymer, (b) pressing the hair with a pressing device including one or more surfaces, and (c) forming a secondary nanostructured surface pattern on the hair that is complementary to the primary nanostructured surface pattern on the one or more surfaces of the pressing device. The secondary nanostructured surface pattern diffracts light into dispersed colors that are visible on the hair. The section of the hair is pressed with the pressing device for from about 1 to 55 seconds. The polymer has a glass transition temperature from about 55 C to about 90 C. The one or more surfaces include a primary nanostructured surface pattern.

Devices for providing UV light are also known in the art. US20040206368A1 mentions a device used for straightening hair. US20040206368A1 states that the "device includes a handle 40 and a flat transparent plate 42... [and the] plate can be passive and merely transmit light of the proper radiation generated by a UV lamp or other light source, or active and generate and emit light 41 from inside the plate 42 as described...for the light-emitting hair curler".

However, there are needs for further and more improved methodologies for providing colour effects on fibres. Indeed, such methodologies are desired in the context of fibres since the use of colorants and other conventional techniques are not always desired.

### SUMMARY OF THE INVENTION

A first aspect relates to a method for providing visual effects on fibres, wherein the method comprises:
(a) providing fibres coated with a composition and applying a forming means onto the fibres, wherein the forming means orders the composition into a pattern, and wherein the forming means is a conductor; and then
(b) raising the temperature of the forming means to a temperature of from 40°C to 150°C for up to 15 minutes; and then
(c) removing the forming means from the fibres;
wherein the composition comprises monomer(s) and optionally a cosmetically acceptable carrier;
wherein the monomer(s) are capable of forming a polymer after exposure to heat;
and wherein the monomer(s) are selected from the group consisting of acrylates monomers, methacrylate monomers, acrylamide monomers, methacrylamide monomers, styrene monomers, vinyl pyrolidinone monomers, vinylpyrrolidone monomers, and mixtures thereof;
and wherein the monomer(s) have a molecular weight in the range from 100 g/mol to 5000 g/mol;
and wherein the monomer(s) have a functionality of between 1 and 100;
and wherein the composition has a kinematic viscosity of from 0.5 cSt to 1500 cSt, measured at 23°C.

A second aspect relates to a kit for providing visual effects on fibres, wherein the kit comprises:
i. i. A composition comprising monomer(s) and optionally a cosmetically acceptable carrier; wherein the monomer(s) are capable of forming a polymer after exposure to heat; and wherein the monomer(s) are selected from the group consisting of acrylates monomers, methacrylate monomers, acrylamide monomers, methacrylamide monomers, styrene monomers, vinyl pyrolidinone monomers, vinylpyrrolidone monomers, and mixtures thereof; and wherein the monomer(s) have a molecular weight in the range from 100 g/mol to 5000 g/mol; and wherein the monomer(s) have a functionality of between 1 and 100; and wherein the composition has a kinematic viscosity of from 0.5 cSt to 1500 cSt, measured at 23°C;
ii. A device for applying the composition onto fibres;
iii. A forming means;
iv. A source of heat.

### BRIEF DESCRIPTION OF THE DRAWINGS

The accompanying drawings, which are incorporated in and form a part of the specification, illustrate the embodiments of the present invention and, together with the description, serve to explain the principles of the invention. In the drawings:
Fig. 1 shows hair following treatment with a method for providing visual effects as described herein.
Fig. 2 shows a sawtooth pattern and lambda and A distances.
Fig. 3 shows an electron micrograph showing lambda distances.

### DETAILED DESCRIPTION OF THE INVENTION

### General and definitions

In this document, including in all embodiments of all aspects of the present invention, the following definitions apply unless specifically stated otherwise. All percentages are by weight (w/w) of the total composition. All ratios are weight ratios. "wt%" means percentage by weight. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams". "Ex." means "example". All amounts as they pertain to listed ingredients are based on the active level ('solids') and do not include carriers or by-products that may be included in commercially available materials. Herein, "comprising" means that other steps and other ingredients can be in addition. "Comprising" encompasses the terms "consisting of" and "consisting essentially of". The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition. For example, if the composition comprises from 1% to 5% fatty alcohol, then a composition comprising 2% stearyl alcohol and 1% cetyl alcohol and no other fatty alcohol, would fall within this scope.

"Substantially free from" or "substantially free of" as used herein means less than about 1%, less than about 0.8%, less than about 0.5%, less than about 0.3%, or about 0%, by total weight of the composition or formulation.

"Hair," as used herein, means mammalian hair including scalp hair, facial hair and body hair, more preferably hair on the human head and scalp. "Hair shaft" means an individual hair strand and may be used interchangeably with the term "hair." "Internal region of the hair shaft," as used herein, means any non-surface portion of the hair shaft, including the inner portion of the cuticle, underneath the cuticle and the cortex. "Non-surface portion" may be understood to mean that portion of the hair that is not in direct contact with the outside environment.

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Monomer," as used herein, means a discrete, non-polymerised chemical moiety capable of undergoing polymerisation. "Ethylenic monomer," as used herein, means a monomer that contains an olefinic carbon-carbon double bond (C=C) and is capable of undergoing polymerisation.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

"Separately packaged," as used herein, means any form of packaging that prevents one composition or formulation from coming into physical contact, or admixing, with a second composition or formulation.

"Kit," as used herein, means a packaging unit comprising a plurality of components i.e. a kit of parts. An example of a kit is, for example, a first composition and a separately packaged second composition. Another kit may comprise application instructions comprising a method and a composition/formulation.

### Chemical mechanism of how the monomer polymerises and causes the pattern on hair

The present invention provides a method for providing visual effects on fibres, such as human hair. A compositon comprising monomer(s) can conform to the shape of a forming means (e.g. a film) having nanostructured steric patterns that give rise to colour effects upon interaction with light. The nano-structured steric pattern is created (molded) into the composition when it is sandwiched in between the fibers and the forming means. Heat which passes through the forming means, causes the monomers to polymerise and harden in the reversed conformation (pattern) of the forming means. Upon removal of the forming means, the hair gives rise to coloured effects that occur from light interactions with the nanostructured patterning on the hardened polymer.

### Advantages of the invention versus what was previously available

By using monomers instead of a polymer composition directly, the created polymer is less likely to deform vs conventional heated polymer solutions used for the embossing process. Indeed, in conventional processes there is no polymerisation in that a monomer solution is used, but instead a polymer composition is used directly. Employment of a composition comprising monomer(s) has the advantage that the composition is less viscous versus polymer composiitons and therefore this lower viscosity means that the composition can be used to cover a greater surface area. In view of the relatively low molecular weight of the monomer(s) versus conventional polymers means that the monomer is more able to penetrate into the fibres (e.g. into the hair shaft) and thus adheres better to the fibres. Another advantage is that there are a number of commercially available films available that can be re-applied to the present invention as forming means from their use in packaging processes.

Furthermore, the present invention provides an alternative to conventional dyeing, embossing and pigment methodologies, which may be permanent or provide unsightly residues, staining and/or rough feel on fibres.

### 1^{st} Aspect: Method

The first aspect relates to a method for providing visual effects on fibres. In at least one embodiment, the fibres are keratin fibres, such as human keratin fibres. In at least one embodiment, the fibres are keratin fibres attached to a human scalp. In at least one embodiment, the fibres are keratin fibres from a mammal. In at least one embodiment, the fibres are attached to a mannequin. In at least one embodiment, the fibres are a swatch of fibres. In at least one embodiment, the fibres are wool fibres, fibres from a non-human mammal or synthetic fibres. In at least one embodiment, the fibres are a component of a non-woven material. In at least one embodiment, the fibres comprise polypropylene, polyethylene terephthalate, polyester, or combinations thereof. In at least one embodiment, the method is for providing a visual effects on a material comprising woven fibres. In at least one embodiment, the fibres are cotton fibres.

In at least one embodiment, where the method is practised on a living mammal, the skin of the living mammal is protected from the heat. In at least one embodiment, where the method is performed by a human (e.g. a hair stylist).

In at least one embodiment, the method does not employ the application of UV light.

### Composition

The method employs a composition. Step (a) of the method relates to (a) providing fibres coated with a composition. The composition comprises monomer(s) and optionally a cosmetically acceptable carrier.

In at least one embodment, the composition is a liquid. In at least one embodment, the composition is a sprayable liquid.

In at least one embodment, the composition is substantially free of an organometallic compound.

### Monomer

The composition comprises monomer(s). By "monomer(s)" it is meant that the composition may comprise only one type of monomer, or it may comprise a plurality of different monomer chemistries i.e. "monomers". For example, the composition may comprise two types of monomers and therefore upon polymerization, a copolymer results. The monomer(s) are capable of forming a polymer after exposure to electromagnetic radiation having a wavelength of from 300 nm to 750 nm. The monomer(s) are selectred from the group consisting of acrylates monomers, methacrylate monomers, acrylamide monomers, methacrylamide monomers, styrene monomers, vinyl pyrolidinone monomers, and mixtures thereof.

The monomer(s) have a functionality of between 1 and 100. By "functionality", the valency of the monomer is meant i.e. the number of reactive sites that are available in the monomer for reacting with other compounds. For example, a bi-functional monomer is able to act as a crosslinking agent because it has two reactive sites. In at least one embodiment, the monomer(s) have a functionality of between 1 and 10, or from 2 and 5.

In at least one embodiment, the monomer is a radically polymerisable monomer. In at least one embodiment, the monomer comprises acrylate, methacrylate, or styrene chemistry, or is a derivative thereof. In at least one embodiment, the monomer is an ethylenic monomer.

The monomer(s) have a molecular weight in the range from 100 to 5000 g/mol. In at least one embodiment, the monomer has a molecular weight of 900 g/mol or less, or has a molecular weight of 500 g/mol or less. In at least one embodiment, the monomer has a molecular weight of at least 110 g/mol.

In at least one embodiment, the monomer is in crystalline form prior to addition into the composition. In at least one embodiment, the monomer is in liquid form prior to addition into the composition.

In at least one embodiment, the monomer is selected from the group consisting of monofunctional acrylic monomers, bi- or poly-functional vinyl monomers, hydrophilic monomers, non-ionic hydrophobic monomers, monomers having at least one carboxylic function, cationic monomers, and combinations thereof.

In at least one embodiment, the composition comprises at least one styrene monomer. In at least one embodiment, the monomer is a crosslinking agent. A crosslinking agent has the advantage that following polymerisation, the unit can be crosslinked to create a crosslinked polymer.

In at least one embodiment, the monomer is a mixture of at least two different monomers, or at least three different monomers, or at least four different monomers, or at least five different monomers, or at least six different monomers, or at least seven different monomers. In at least one embodiment, the composition comprises less than ten different monomers, or less than nine different monomers, or less than eight different monomers, or less than seven different monomers, or less than six different monomers.

In at least one embodiment, the composition comprises a mixture of different monomers wherein the composition comprises an anionic monomer, a non-ionic monomer and a monomer having amide chemistry.

In at least one embodiment, the compositon comprises an ethylenic monomer. In at least one embodiment, the ethylenic monomer comprises a vinyl group. "Vinyl group" as used herein, means H₂C=CH-R. In at least one embodiment, the ethylenic monomer comprises an acrylate group or a methacrylate group. "Acrylate group" as used herein, means H₂C=CH-C(O)O-R. Acrylic acid, for example, comprises an acrylate group since R is hydrogen. "Methacrylate group" as used herein means H₂C=C(CH₃)-C(O)O-R.

In at least one embodiment, the composition comprises an amide monomer. Amides have the advantage that they adhere excellently to fibres.

In at least one embodiment, the composition comprises a bi- or poly-functional vinyl monomer. In at least one embodiment, the bi- or poly-functional vinyl monomer is selected from the group consisting of allyl methacrylate, triethylene glycol dimethacrylate, ethylene glycol dimethacrylate, diethylene glycol dimethacrylate, aliphatic or aromatic urethane diacrylates, difunctional urethane acrylates, ethoxylated aliphatic difunctional urethane methacrylates, aliphatic or aromatic urethane dimethacrylates, epoxy acrylates, epoxymethacrylates, tetraethylene glycol dimethacrylate, polyethylene glycol dimethacrylate, 1,3 butylene glycol diacrylate, 1,4-butanediol dimethacrylate, 1,4-butaneidiol diacrylate, diethylene glycol diacrylate, 1,6 hexanediol diacrylate, 1,6 hexanediol dimethacrylate, neopentyl glycol diacrylate, polyethylene glycol diacrylate, tetraethylene glycol diacrylate, triethylene glycol diacrylate, 1,3 butylene glycol dimethacrylate, tripropylene glycol diacrylate, ethoxylated bisphenol diacrylate, ethoxylated bisphenol dimethylacrylate, dipropylene glycol diacrylate, alkoxylated hexanediol diacrylate, alkoxylated cyclohexane dimethanol diacrylate, propoxylated neopentyl glycol diacrylate, trimethylolpropane trimethacrylate, trimethylolpropane triacrylate, pentaerythritol triacrylate, ethoxylated trimethylolpropane triacrylate, propoxylated trimethylolpropane triacrylate, propoxylated glyceryl triacrylate, ditrimethylolpropane tetraacrylate, dipentaerythritol pentaacrylate, ethoxylated pentaerythritol tetraacrylate, and mixtures thereof.

In at least one embodiment, the composition comprises a bi-functional vinyl monomer.

In at least one embodiment, the composition comprises a monofunctional monomer. In at least one embodiment, the monofunctional monomer is selected from the group consisting of acrylic monomers such as C₁-C₂₀ alkyl acrylates, C₁-C₂₀ alkyl methacrylates, C₁-C₂₀ alkyl-aryl acrylates, C₁-C₂₀ alkyl-aryl methacrylates, C₁-C₂₀ alkyl-aryl and didalkyl-aryl acrylamides, C₁-C₂₀ alkyl-aryl and dialkyl-aryl methacrylamides, styrenics, and mixtures thereof.

In at least one embodiment, the monomer is a hydrophilic monomer. In at least one embodiment, the monomer is a hydrophilic monomer and is selected from the group consisting of α,β-ethylenically unsaturated acids, α,β-ethylenically unsaturated amides, α,β-ethylenically unsaturated monoalkyl amides, α,β-ethylenically unsaturated dialkyl amides, α,β-ethylenically unsaturated monomers bearing a water-soluble polyoxyalkylene segment of the poly(ethylene oxide) type, α,β-ethylenically unsaturated monomers which are precursors of hydrophilic units or segments, vinylpyrrolidones, α,β-ethylenically unsaturated monomers of the ureido type, and mixtures thereof.

In at least one embodiment, the monomer is a non-ionic hydrophobic monomer. In at least one embodiment, the non-ionic hydrophobic monomer is selected from the group consisting of: vinylaromatic monomers, vinyl halides, vinylidene halides, C₁-C₁₂ alkylesters of α,β-monoethylenically unsaturated acids, vinyl esters of saturated carboxylic acids, allyl esters of saturated carboxylic acids, α,β-monoethylenically unsaturated nitriles containing from 3 to 12 carbon atoms, α-olefins, conjugated dienes, and mixtures thereof.

In at least one embodiment, the anionic monomer is selected from the group consisting of: monomers having at least one carboxylic function, for instance α,β-ethylenically unsaturated carboxylic acids or the corresponding anhydrides, monomers that are precursors of carboxylate functions, monomers having at least one sulfate or sulfonate function, monomers having at least one phosphonate or phosphate function, esters of ethylenically unsaturated phosphates, and mixtures thereof. In at least one embodiment, the anionic monomer is derived from an anionic monomer selected from the group consisting of: acrylic acid, methacrylic acid, 2-acrylamido-2-methylpropane sulfonic acid, carboxyethyl acrylate, and mixtures thereof.

In at least one embodiment, the monomer is a cationic monomer. In at least one embodiment, the cationic monomer is selected from the group consisting of: acryloyl- or acryloyloxyammonium monomers, 1-ethyl-2-vinylpyridinium or 1-ethyl-4-vinylpyridinium bromide, chloride or methyl sulfate, N,N-dialkyldiallylamine monomers, polyquaternary monomers, N,N-(dialkylamino-ω-alkyl)amides of α,β-monoethylenically unsaturated carboxylic acids, α,β-monoethylenically unsaturated amino esters, vinylpyridines, vinylamine, vinylimidazolines, monomers that are precursors of amine functions which give rise to primary amine functions by simple acid or base hydrolysis, and mixtures thereof. In at least one embodiment, the cationic monomer is derived from methacrylamidopropyl trimethyl ammonium chloride (MAPTAC). In at least one embodiment, the cationic monomer is derived from diallyldimethylammonium chloride (DADMAC). In at least one embodiment, the cationic monomer is derived from 2-hydroxy-N1-(3-(2((3-methacrylamidopropyl)dimethylammino)-acetamido)propyl)-N1, N1, N3, N3, N3-pentamethylpropane-1,3-diaminium chloride.

In at least one embodiment, the monomer is selected from the group consisting of trimethylolpropane triacrylate, pentaerythritol triacrylate, ethylene glycol dimethacrylate, polyethylene glycol dimethacrylate, 1,3-butylene glycol diacrylate, 1,6-hexanediol diacrylate, tripropylene glycol diacrylate, propoxylated neopentyl glycol diacrylate, pentaerythritol tetra-acrylate, dipentaerythritol pentaacrylate, butyl acrylate, 2-ethylhexyl methacrylate, butyl methacrylate, and mixtures thereof.

In at least one embodiment, the monomer is selected from the group consisting of pentaerythritol triacrylate, ethylene glycol dimethacrylate, 1,3-butylene glycol diacrylate, propoxylated neopentyl glycol diacrylate, pentaerythritol tetra-acrylate, and mixtures thereof. In at least one embodiment, the composition comprises a mixture of pentaerythritol triacrylate, ethylene glycol dimethacrylate, 1,3-butylene glycol diacrylate, propoxylated neopentyl glycol diacrylate, and pentaerythritol tetra-acrylate. In at least one embodiment, the composition comprises a monomer mixture consisting of pentaerythritol triacrylate, ethylene glycol dimethacrylate, 1,3-butylene glycol diacrylate, propoxylated neopentyl glycol diacrylate, and pentaerythritol tetra-acrylate.

In at least one embodiment, the monomer is selected from the group consisting of trimethylolpropane triacrylate, 1,6-hexanediol diacrylate, dipentaerythritol pentaacrylate, butyl acrylate, and mixtures thereof. In at least one embodiment, the composition comprises a mixture of trimethylolpropane triacrylate, 1,6-hexanediol diacrylate, dipentaerythritol pentaacrylate, and butyl acrylate. In at least one embodiment, the mixture of monomers consisting of trimethylolpropane triacrylate, 1,6-hexanediol diacrylate, dipentaerythritol pentaacrylate, and butyl acrylate.

In at least one embodiment, the monomer is selected from the group consisting of pentaerythritol tetra-acrylate, 2-ethylhexyl methacrylate, and their mixture. In at least one embodiment, the composition comprises a mixture of monomers consisting of pentaerythritol tetra-acrylate, 2-ethylhexyl methacrylate.

In at least one embodiment, the monomer is selected from the group consisting of tripropylene glycol diacrylate, 2-ethylhexyl methacrylate, and their mixtures. In at least one embodiment, the composition comprises a mixture of monomers consisting of tripropylene glycol diacrylate and 2-ethylhexyl methacrylate.

In at least one embodiment, the monomer is selected from the group consisting of trimethylolpropane triacrylate, butyl methacrylate, and their mixture. In at least one embodiment, the composition comprises a mixture of monomers consisting of trimethylolpropane triacrylate and butyl methacrylate.

In at least one embodiment, the monomer is selected from the group consisting of pentaerythritol triacrylate, polyethylene glycol dimethacrylate, and their mixture. In at least one embodiment, the composition comprises a mixture of monomers consisting of pentaerythritol triacrylate and polyethylene glycol dimethacrylate.

In at least one embodiment, the monomer is selected from the group consisting of tripropylene glycol diacrylate, dipentaerythritol pentaacrylate, and their mixture. In at least one embodiment, the composition comprises a mixture of monomers consisting of tripropylene glycol diacrylate and dipentaerythritol pentaacrylate.

Where composition comprises a mixture of monomers consisting of monomers X, Y, Z, then this means that no further monomers other than monomers X, Y and Z are present in the composition because the "consisting of" language excludes the presence of any other monomer.

### Polymerization inhibitor

In at least one embodiment, the composition comprises a polymerisation inhibitor. In at least one embodiment, the composition comprises polymerisation inhibitor in an amount of from 1 milligram to 1000 milligram per kilogram of total monomer.

In at least one embodiment, the polymerisation inhibitor is an anisole compound. In at least one embodiment, the polymerisation inhibitor is selected from the group consisting of: 2-tert-butyl-4-hydroxy-anisole, 3-tert-butyl-4-hydroxy-anisole, and mixtures thereof.

Regarding the anisole compound as polymerisation inhibitor, it is believed that upon the formation of a carbon radical in place of the -CH=CH₂ group on an ethylenic monomer, for example, the the polymerisation inhibitor is able to donate a proton from its hydroxyl group to the radical on the ethylenic monomer. The resulting monomer is unable to polymerise since a radical is no longer present. It is also believed that the butyl group on the polymerisation inhibitor acts as an electron-donating (+I) group able to stabilise the resulting radical in the phenyl ring. This is explained more precisely hereinafter. Formula I shows 3-*tert*-butyl-4-hydroxy-anisole (CAS No.: 121-00-6), wherein the methoxy group is represented by "OMe" and the tert-butyl group by "Bu-t". As used herein, the carbon-1 position for the herein mentioned anisole compound as polymerisation inhibitor is the carbon substituted with the methoxy group.

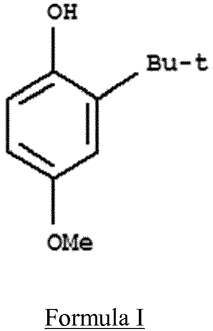

An example of an ethylenic monomer is 3-sulfopropacrylate (3-SPA). It will, over time, generate radicals at the vinyl position that can react with another monomer and could prematurely polymerise and thus preferably is to be stabilized to avoid premature polymerisation. Ethylenically unsaturated monomers such as 3-SPA can be abbreviated "R" (see Formula II): 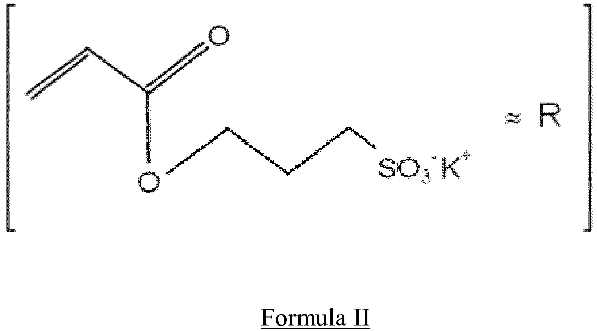 Once an ethylenic monomer radical (R) is created, it reacts with inhibitor the polymerisation inhibitor. Inhibitors generally form stabile, thus unreactive radicals, and hence prevent the monomer radical from premature auto-polymerisation. When reacting with the polymerisation inhibitor, the monomer radical abstracts hydrogen from the hydroxyl group, and thus gets regenerated (see Scheme I): 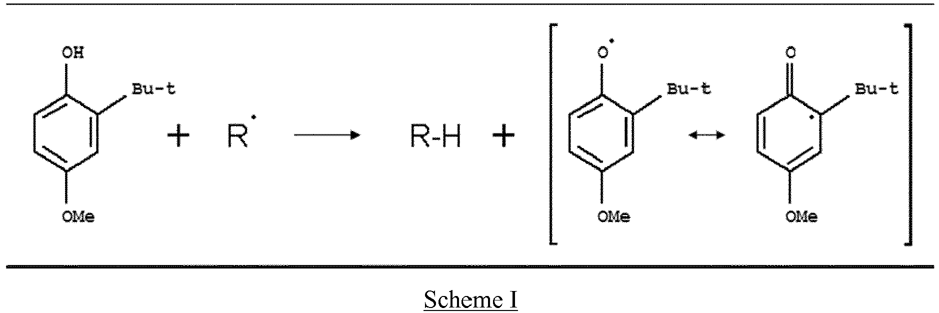 Without wanting to be bound by theory it is believed that the polymerisation inhibitor being 2-tert-butyl-4-hydroxy-anisole, 3-tert-butyl-4-hydroxy-anisole, or a mixtures thereof is a more effective inhibitor for the polymerisation of ethylenically unsaturated monomers compared to 4-methoxy phenol, for example, because it forms more stable intermediates. The tert-butyl group of the polymerisation inhibitor has a positive electron inducing effect for the ring radical formed upon hydrogen abstraction, thus is more likely to form for the polymerisation inhibitor than for 4-methoxy phenol (see Scheme II).

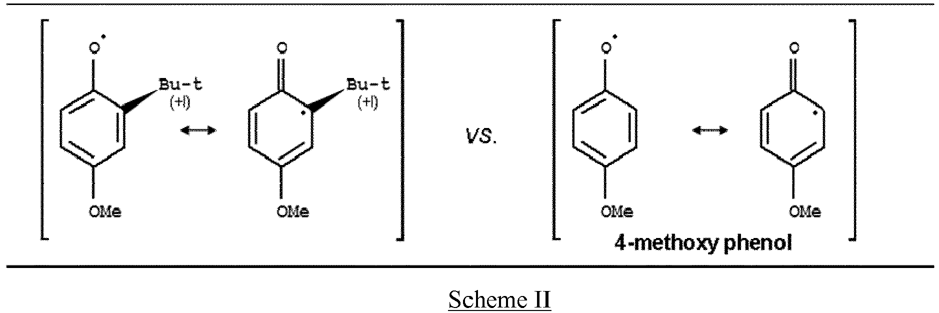

In at least one embodiment, the polymerisation inhibitor is nitrobenzene or diphenyl picryl hydrazyl (DPPH). For example Irganox® from BASF can be used. Irganox® 1330, for example, is a Tris-BHT Mesitylene compound. Ethanox from SI Group is an non-coloring, odourless antioxidant. For example, ETHANOX 330 or 330G can be used.

### Cosmetically Acceptable Carrier

The composition optionally comprises a cosmetically acceptable carrier. In at least one embodiment, the composition comprises a cosmetically acceptable carrier. In at least one embodiment, the cosmetically acceptable carrier is any carrier suitable for formulating the active agent into a composition being suitable for application onto hair. In at least one embodiment, the cosmetically acceptable carrier is selected from either an aqueous medium or an aqueous-alcoholic medium. In at least one embodiment, when the carrier is an aqueous-alcoholic carrier, this carrier comprises water and an alcohol. Alcohol has the advantage that it can influence the viscosity of a wider spectrum of composition components, such as polymers. In at least one embodiment, the alcohol is selected from the group consisting of: ethanol, isopropanol, propanol, and mixtures thereof. In at least one embodiment, when the carrier is an aqueous carrier, this carrier consists essentially of water and is substantially free of alcohol. In at least one embodiment, the composition comprises a safe and effective amount of cosmetically acceptable carrier. In at least one embodiment, the composition comprises from 0.1% to 99%, or from 1% to 98%, or from 10% to about 97%, or from 30% to 95% water.

In at least one embodiment, the composition is substantially free of alcohol, such as volatile alcohols (e.g. ethanol, isopropanol, propanol). No alcohol is advantageous because a no-alcohol composition has reduced odour and/or flammability issues.

In at least one embodiment, the cosmetically acceptable carrier is an oily compound. In at least one embodiment, the oily compound is selected from the group consisting of cyclic silicones and volatile hydrocarbons. Cyclic silicones are available from Dow Corning. In at least one embodiment, the cyclic silicone has from at least about 3 silicone atoms or from at least about 5 silicone atoms but no more than about 7 silicone atoms or no more than about 6 silicone atoms. In at least one embodiment, the cyclic silicone conforms to the formula: 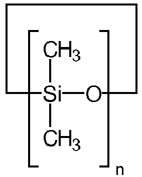 wherein n is from 3 or from 5 but no more than 7 or no more than 6. In at least one embodiment, the cyclic silicone have a kinematic viscosity of less than about 10 cSt at 23°C. A Suitable cyclic silicones for use herein include, but are not limited to, Cyclomethicone D5 (commercially available from G.E. Silicones).

Volatile hydrocarbons e.g. Isopar can be obtained from ExxonMobil Petroleum and Chemical. In at least one embodiment, the oily compiound is a mineral oils. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

### Viscosity

The composition has a kinematic viscosity of from 0.5 cSt to 1500 cSt, measured at 23°C. "Viscosity" can mean dynamic viscosity (measured in mPa•s) or kinematic viscosity (measured in centistokes, cSt) of a liquid at 23°C and ambient conditions. Dynamic viscosity may be measured using a rotational viscometer, such as a Brookfield Dial Reading Viscometer Model 1-2 RVT available from Brookfield Engineering Laboratories (USA) or other substitutable model as known in the art. Typical Brookfield spindles which may be used include, without limitation, RV-7 at a spindle speed of 20 rpm, recognizing that the exact spindle may be selected as needed by one skilled in the art. Kinematic viscosity may be determined by dividing dynamic viscosity by the density of the liquid (at 23°C and ambient conditions), as known in the art.

The viscosity is useful in view of enabling the composition to be easily applied to the fibres - e.g. spread evenly onto the fibres. Viscosity can be influenced by the level of cosmetically acceptable carrier in the composition. For example, where there are e.g. around 5 or 6 different functional monomers in the composition, the composition could be fairly viscous and would likely need a cosmetically acceptable carrier to reduce the viscosity.

In at least one embodiment, the composition has a kinematic viscosity of from 1 cSt to 1000 cSt. In at least one embodiment, the composition has a kinematic viscosity of from 1.5 cSt to 500 cSt, or from 2 cSt to 350 cSt, or from 2.5 cSt to 200 cSt, or from 3 cSt to 150 cSt, measured at 23°C. 1 centistoke (cSt) is equal to 1 x 10⁻⁶ m²/s).

In at least one embodiment, the composition has a dynamic viscosity of from 1 mPa·s to 5000 mPa·s. In at least one embodiment, the composition has a viscosity of from 2 mPa·s to 400 mPa·s, or from 3 mPa·s to 100 mPa·s. Alternatively, in at least one embodiment, the composition has a dynamic viscosity of from 30 mPa·s to 250 mPa·s, or from 100 mPa·s to 200 mPa·s.

This viscosity range is useful in view of helping to prevent the composition from dripping. Furthermore, when the viscosity is too high, the composition cannot easily be mixed e.g. with the cosmetically acceptable carrier, where present.

### Volatility

In at least one embodiment, the composition is substantially free of compounds having a vapor pressure below 0.01 mmHg, or below 0.001 mm Hg, measured at 23°C and 1 atm. Low volatility has the advantage that the composition has reduced odour and also potentially may have a safer safety profile.

### pH

In at least one embodiment, the composition has a pH of from pH 5 to pH 10, or from pH 6 to 9, or from pH 7 or pH 8, or from pH 6.5 to pH 7.5. It is advantageous for the pH to be as close to neutral as possible - for example in view of reduced likelihood of fibre damage. Moreover, a pH that is compatible with polymerisation is useful.

### Photoinitiator

In at least one embodiment, the composition comprises a photoinitiator. A photoinitiator has the advantage that a free radical is formed, which helps initiate the polymerization. An additional benefit is that less intense electromagnetic radiation is required in the method when the composition comprises a photoinitiator. For example, a UV light induced free-radical initiating step comprises exposing the composition with UV light for a time sufficient to decompose the UV-reactive photoinitiator thereby initiating the polymerisation of monomers.

In at least one embodiment, the composition comprises from 0.1% to 5%, or from 0.5% to 2% photoinitiator.

In at least one embodiment, the photoinitiator is an organic photoinitiator. Organic photoinitiators have the advantage over inorganic photoinitiators (e.g. titanium dioxide) in that specific wavelengths can be chosen based on the organic photoinitiator structure. Organic photoinitiators are also advantageous in view of being less aggressive towards the oxidation of organic compounds.

In at least one embodment, the photoinitiator is an organic aromatic compound. In at least one embodment, the photoinitiator is an alpha-hydroxyketone. In at least one embodment, the photoinitiator is selected from the group consisting of: phenyl ketones, benzoinethers, benzoil ketals, azo initiators, and mixtures thereof. In at least one embodment, the polymerisation is accomplished by exposing the composition to UV light.

In at least one embodment, the photoinitiator is selected from the group consisting of: thixoanthone, riboflavin, eosin Y, phloxine B, and mixtures thereof. Thixoanthone has a preferred absorption wavelength of about 390 nm. Riboflavin has a preferred absorption wavelength of about 420 nm. Eosin Y has a preferred absorption wavelength of about 510 nm. Phloxine B has a preferred absorption wavelength of about 560 nm. Thixoanthone, riboflavin, eosin Y, and phloxine B are depicted below from left to right:

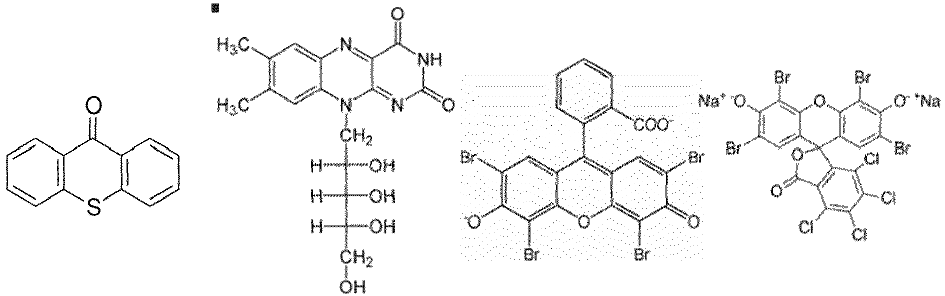

Irgacure® 184 available from BASF, Ludwigshafen, Germany is a suitable phenyl ketone organic photoinitiator and has the chemical formula: 1-hydroxy-cyclohexyl-phenyl-ketone. Darocure® 1173 available from BASF, Ludwigshafen, Germany (previously Ciba Specialty Chemicals Inc) is a suitable phenyl ketone organic photoinitiator and has the chemical formula: 2-hydroxy-2-methyl-1-phenyl-propan-1-one.

### Perfume

In at least one embodiment, the composition comprises perfume. In at least one embodiment, the composition comprises from 0.001% to 2% perfume. Perfume can provide an enhanced user experience by making the composition smell pleasant and/or invoke emotions tailored to the visual effects on the fibres - such as relaxing or exciting smells.

In at least one embodiment, the composition is substantially free of perfume and/or fragrance. Some consumers prefer perfume-free compositions and therefore this can also be advantageous.

The perfume may also be provided via an encapsulated perfume i.e. a perfume provided inside a microcapsule. In at least one embodiment, the composition comprises encapsulated perfume.

In at least one embodiment, the microcapsule features friction-triggered release technology i.e. the contents of the microcapsule is released upon exposing the microcapsule friction. Said friction could be the action of sponging the composition according to the present invention onto the hair or combing the hair after the composition has been applied. In at least one embodiment, the microcapsule is a friable microcapsule. A friable microcapsule is configured to release the core material when the outer shell is ruptured. In at least one embodiment, the microcapsule comprises a shell made from a synthetic polymeric material. In at least one embodiment, the microcapsule comprise a core material and a shell surrounding the core material, wherein the shell comprises: a plurality of amine monomers selected from the group consisting of aminoalkyl acrylates, alkyl aminoalkyl acrylates, dialkyl aminoalykl acrylates, aminoalkyl methacrylates, alkylamino aminoalkyl methacrylates, dialkyl aminoalykl methacrylates, tertiarybutyl aminethyl methacrylates, diethylaminoethyl methacrylates, dimethylaminoethyl methacrylates, dipropylaminoethyl methacrylates, and mixtures thereof; and a plurality of multifunctional monomers or multifunctional oligomers. In at least one embodiment, the shell consists of a polyacyrylate material, such as a polyacrylate random copolymer. In at least one embodiment, the microcapsule features moisture-triggered release technology i.e. the contents of the microcapsule is released upon contact with moisture. In at least one embodiment, the microcapsule comprises cyclic oligosaccharides, or the microcapsule matrix or shell is made from cyclic oligosaccharides. "Cyclic oligosaccharide" means a cyclic structure comprising six or more saccharide units. In at least one embodiment, the cyclic oligosaccharides have six, seven, or eight saccharide units or combinations thereof. It is common in the art to refer to six, seven and eight membered cyclic oligosaccharides as α, β, and γ, respectively. In at least one embodiment, the cyclic oligosaccharides are selected from the cyclodextrins: methyl-α-cyclodextrins, methyl-β-cyclodextrins, hydroxypropyl-α-cyclodextrins, hydroxypropyl-β-cyclodextrins, and mixtures thereof. The cyclodextrins may be in the form of particles. The cyclodextrins may also be spray-dried.

In at least one embodiment, the perfume is an animal fragrance or a plant fragrance. In at least one embodiment, the animal fragrance is selected from consisting of musk oil, civet, castoreum, ambergris, and mixtures thereof. In at least one embodiment, the plant fragrance is selected from consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof. In at least one embodiment, the perfume is selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

### Preservative

In at least one embodiment, the composition comprises at least one preservative and/or a mixture of preservatives. In at least one embodiment, the preservative and/or mixture of preservatives is/are active against gram negative bacteria, *Staphylococcus aureus* and *Candida albicans.* In at least one embodiment, the composition comprises 2-phenoxyethanol and/or phenylmethanol. In at least one embodiment, the composition comprises 2-phenoxyethanol.

In at least one embodiment, the composition is substantally free of preservative. Indeed, sometimes consumers prefer preservative-free compositions.

In at least one embodiment, the composition comprises from 0.01% to 5% preservative, or from 0.1% to 2%, or from 0.5% to 1.5% preservative. In at least one embodiment, the composition comprises from 100 ppm to 500 ppm preservative. In at least one embodiment, the preservative is selected from the group consisting of benzyl alcohol, phenoxyethanol, and mixtures thereof. In at least one embodiment, the composition is substantially free of esters of parahydroxybenzoic acid. Esters of parahydroxybenzoic acid are commonly known as parabens. Parabens are not preferred by some consumers. In at least one embodiment, the composition is substantially free of isothiazolinone compounds. In at least one embodiment, the composition is substantially free of benzoate compounds. Benzoate compounds may not preferred in view of the potential for instability and/or precipitation of the composition. In at least one embodiment, the composition is substantially free of 1,3-bis(hydroxymethyl)-5,5-dimethylimidazolidine-2,4-dione.

### Pigments

In at least one embodiment, the composition comprises pigment. In at least one embodiment, the pigments are coloured pigments which impart colour effects to the product mass or to the hair, or they may be lustre effect pigments which impart desirable and aesthetically pleasing lustre effects to the composition or to the keratin fibres. The colour or lustre effects on the hair are preferably temporary, i.e. they last until the next hair wash and can be removed again by washing the hair with customary shampoos.

In at least one embodiment, the composition is substantally free of pigment. Indeed, sometimes consumers prefer pigment-free compositions.

In at least one embodiment, the composition comprises pigment having a D₅₀ particle diameter of from 5 micron to 60 micron. Particle diameter is represented by D₅₀, which is the median diameter by volume. D₅₀ is measured with a Malvern Mastersizer 2000, which is a laser diffraction particle sizer and it is measured according to ISO 13320:2009(en) with Hydro 2000G or Hydro 2000S where the dispersant is water or ethanol. Detection range is from 0.02 micron to 2000 micron. D₅₀ is expressed as x₅₀ in ISO 13320:2009(en). Laser diffraction measures particle size distributions by measuring the angular variation in intensity of light scattered as a laser beam passes through a dispersed particulate sample analyser and the particle size is reported as a volume equivalent sphere diameter. A discussion of calculating D₅₀ is provided in Barber et al, Pharmaceutical Development and Technology, 3(2), 153-161 (1998), which is incorporated herein by reference.

In at least one embodiment, the composition comprises pigment having a D₅₀ particle diameter of from 10 micron to 40 micron. In at least one embodiment, the pigments are present in the composition in undissolved form. In at least one embodiment, the composition comprises from 0.01% to 25%, or from 0.1% to 20% pigment, or from 1% to 15%, or from 4% to 10% pigment. The pigments are colorants which are virtually insoluble in the composition, and may be inorganic or organic. Inorganic-organic mixed pigments are also possible. In at least one embodiment, the composition comprises inorganic pigments. The advantage of inorganic pigments is their excellent resistance to light, weather and temperature. In at least one embodiment, the inorganic pigments are of natural origin, and are, for example, derived from material selected from the group consisting of chalk, ochre, umber, green earth, burnt sienna, and graphite. In at least one embodiment, the pigments are white pigments, such as, for example, titanium dioxide or zinc oxide, or are black pigments, such as, for example, iron oxide black, or are coloured pigments, such as, for example, ultramarine or iron oxide red, lustre pigments, metal effect pigments, pearlescent pigments, and fluorescent or phosphorescent pigments. In at least one embodiment, the pigments are coloured, non-white pigments. In at least one embodiment, the pigments are selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments). In at least one embodiment, the pigments are selected from the group consisting of are titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

In at least one embodiment, the pigments are pearlescent and coloured pigments based on mica which are coated with a metal oxide or a metal oxychloride, such as titanium dioxide or bismuth oxychloride, and optionally further colour-imparting substances, such as iron oxides, Prussian blue, ultramarine, and carmine. The colour exhibited by the pigment can be adjusted by varying the layer thickness. Such pigments are sold, for example, under the trade names Rona^{®}, Colorona^{®} Dichrona^{®}, RonaFlair^{®}, Ronastar^{®}, Xirona^{®} and Timiron^{®} all of which are available from Merck, Darmstadt, Germany. For example, Xirona^{®} is a brand for colour travel pigments that display colour shifting effects depending on the viewing angle and are based on either natural mica, SiO₂ or calcium aluminium borosilicate flakes, coated with varying layers of TiO₂. Pigments from the line KTZ® from Kobo Products, Inc., 3474 So. Clinton Ave., So. Plainfield, USA, are also useful herein, in particular the Surface Treated KTZ® Pearlescent Pigments from Kobo. Particularly useful are KTZ® FINE WHITE (mica and TiO₂) having a D₅₀ particle diameter of 5 to 25 micron and also KTZ® CELESTIAL LUSTER (mica and TiO₂, 10 to 60 micron) as well as KTZ® CLASSIC WHITE (mica and TiO₂, 10 to 60 micron). Also useful are SynCrystal Sapphire from Eckart Effect Pigments, which is a blue powder comprising platelets of synthetic fluorphlogopite coated with titanium dioxide, ferric ferrocyanide and small amounts of tin oxide. Also useful is SYNCRYSTAL Almond also from Eckart, which is a beige powder with a copper reflection colour and is composed of platelets of synthetic fluorphlogopite and coated with titanium dioxide and iron oxides. Also useful is Duocrome® RV 524C from BASF, which provides a two colour look via a lustrous red powder with a violet reflection powder due to its composition of mica, titanium dioxide and carmine.

In at least one embodiment, the pigments are organic pigments. In at least one embodiment, the organic pigment is selected from the group consisting of natural pigments sepia, gamboge, bone charcoal, Cassel brown, indigo, chlorophyll and other plant pigments. In at least one embodiment, the synthetic organic pigments are selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

In at least one embodiment, the pigments are selected from the group consisting of iron oxide, titanium dioxide, mica, borosilicate, and combinations thereof. In at least one embodiment, the pigment comprises an iron oxide (Fe₂O₃) pigment. In at least one embodiment, the pigment comprises a combination of mica and titanium dioxide.

### Coloured material

In at least one embodiment, the composition comprises coloured material. In at least one embodiment, the coloured material is particulate in form. In at least one embodiment, the coloured material is selected from the group consisting of coloured fibres, coloured beads, coloured particles such as nano-particles, coloured polymers comprising covalently attached dyes, liquid crystals, particles having diffraction properties, UV absorber and photoprotective substances, pressure- or light-sensitive pigments, and combinations thereof.

In at least one embodiment, the composition is substantally free of coloured material. Indeed, sometimes consumers prefer coloured material-free compositions.

In at least one embodiment, the coloured material is capable of changing colour via a mechanism selected from the group consisting of thermochromism, photochromism, hydrochromism, magnetochromism, electrochromism, piezochromism, chemichromism, mechano-optics. Suitable materials include 3D Magnetic Pigments, Glow Dust, Flourescent Pigments, Thermo Dust, Chameleon Pigments and other colour changing materials from Solar Color Dust (http://solarcolordust.com/).

In at least one embodiment, the composition comprises a photoprotective substance. In at least one embodiment, the composition comprises from 0.01 to 10%, or from 0.1 to 5%, or from 0.2 to 2% photoprotective substance. Useful photoprotective substances are specified in EP1084696A1 from §0036 to §0053, which is incorporated herein by reference. In at least one embodiment, the photoprotective substance is selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, di-butyl-hydroxytoluene (BHT), and mixtures thereof.

In at least one embodiment, the composition comprises from 0.01% to 10%, or from 0.05% to 5% particulate substance. In at least one embodiment, the particulate substances are substances which are solid at room temperature (23°C) and are in the form of particles. In at least one embodiment, the particulate substance is selected from the group consisting of silica, silicates, aluminates, clay earths, mica, and insoluble salts. In at least one embodiment, the particulate substance is selected from the group consisting of insoluble inorganic metal salts, metal oxides, minerals and insoluble polymer particles. In at least one embodiment, the particulate substance is titanium dioxide.

In at least one embodiment, the particulate substance is present in the composition in undissolved, or stably dispersed form, and, following application to the hair and evaporation of the solvent, can deposit on the hair in solid form.

In at least one embodiment, the particulate substance is selected from the group consisting of silica (silica gel, silicon dioxide) and metal salts, in particular inorganic metal salts. In at least one embodiment, the particulate substance is silica. In at least one embodiment, the particulate substance is selected from the group consisting of metal salts such as alkali metal or alkaline earth metal halides, e.g. sodium chloride or potassium chloride; alkali metal or alkaline earth metal sulfates, such as sodium sulfate or magnesium sulfate.

### Hair colouring agent

In at least one embodiment, the composition further comprises a hair colouring agent. In at least one embodiment, the hair colouring agent is a direct dye. In at least one embodiment, the composition comprises a total amount of from 0.001% to 4%, or from 0.005% to 3%, or from 0.01% to 2% direct dye. The presence of a direct dye and the proportion thereof is useful in that it can provide or enhance colouring/dyeing, particularly with regard to intensity.

In at least one embodiment, the composition is substantally free of hair colouring agent. Indeed, sometimes consumers prefer hair colouring agent-free compositions.

In at least one embodiment, the direct dye is selected from the group consisting of nitro dyes to provide a blue colour, nitro dyes to provide a red colour, nitro dyes to provide a yellow colour, quinone dyes, basic dyes, neutral azo dyes, acid dyes, and mixtures thereof. In at least one embodiment, the direct dye is a nitro dye to provide a blue colour. In at least one embodiment, the direct dye is a nitro dye to provide a red colour. In at least one embodiment, the direct dye is a nitro dye to provide a yellow colour. In at least one embodiment, the direct dye is a quinone dye. In at least one embodiment, the direct dye is a basic dye. In at least one embodiment, the direct dye is a neutral azo dye. In at least one embodiment, the direct dye is an acid dye. In at least one embodiment, the direct dye is selected from the group consisting of Acid dyes such as Acid Yellow 1, Acid Orange 3, Acid Black 1, Acid Black 52, Acid Orange 7, Acid Red 33, Acid Yellow 23, Acid Blue 9, Acid Violet 43, Acid Blue 16, Acid Blue 62, Acid Blue 25, Acid Red 4, Basic Dyes such as Basic Brown 17, Basic Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, 4-(3-(4-amino-9,10-dioxo-9,10-dihydroanthracen-1-ylamino)propyl)-4-methylmorpholin-4-ium-methylsulfate, (E)-1-(2-(4-(4,5-dimethylthiazol-2-yl)diazenyl)phenyl)(ethyl)amino)ethyl)-3-methyl-1H-imidazol-3-ium chloride, (E)-4-(2-(4-(dimethylamino)phenyl)diazenyl)-1-methyl-1H-imidazol-3-ium-3-yl)butane-1-sulfonate, (E)-4-(4-(2-methyl-2-phenylhydrazono)methyl)pyridinium-1-yl)butane-1-sulfonate, N,N-dimethyl-3-(4-(methylamino)-9,10-dioxo-4a,9,9a,10-tetrahydroanthracen-1-ylamino)-N-propylpropan-1-aminium bromide, Disperse Dyes such as Disperse Red 17, Disperse Violet 1, Disperse Red 15, Disperse Violet 1, Disperse Black 9, Disperse Blue 3, Disperse Blue 23, Disperse Blue 377, Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No.1, HC Blue No. 14, and Natural dyes such as Annato, Anthocyanin, Beetroot, Carotene, Capsanthin, Lycopene, Chlorophyll, Henna, Indigo, Cochineal.

### Application

Step (a) relates to providing fibres coated with a composition and applying a forming means onto the fibres, wherein the forming means orders the composition into a pattern, and wherein the forming means is a conductor. In at least one embodiment, the forming means is also permeable to UV radiation.

Step (a) encompasses coating fibres with the composition and then applying the forming means onto the fibres. Step (a) also encompasses providing a forming means that is already coated with the composition. In this case, when the coated forming means is applied onto the fibres, the fibres are also coated with the composition. In the latter option, the fibres may not be coated as evenly as desired. Consequently, it is advantageous if step (a) is carried out by:
- Firstly, coating the fibres with the composition, such as by spraying the composition onto the fibres, or by coating the fibres with a device such as a sponge, comb, or brush; and then
- Secondly, applying a forming means onto the fibres, wherein the forming means orders the composition into a pattern, and wherein the forming means is a conductor.

In at least one embodiment, the fibres are coated with the composition using a sponge, comb, or brush. In at least one embodiment, the fibres are coated with the composition using a spray head. In this case, the composition may be provided in a receptable with a spray head and the composition is sprayed onto the fibres. In at least one embodiment, the fibres are coated with the composition using a pump spraying device or an aerosol spraying device.

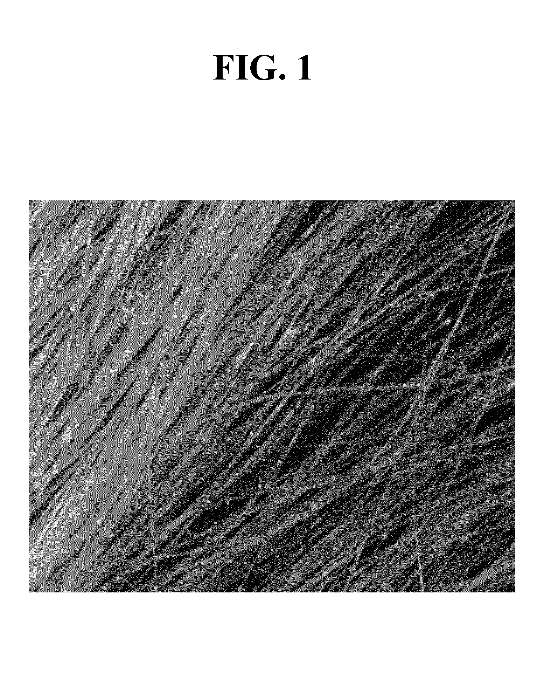

In at least one embodiment, the fibres are coated with the composition using an applicator. Suitable applicators are disclosed in Lund and Smith, EP2002750A1 (published on 17th Dec 2008), particularly Fig. 1 to 4, which are incorporated herein by reference.

In at least one embodment, the composition forms a coating on the fibres. In at least one embodiment, the coating has a mean thickness of from 2 nm to 1000 nm, or from 10 nm to 200 nm, or from 20 nm to 180 nm, or from 30 nm to 100 nm. The mean thickness can be measured with microscopy (SEM). The mean thickness is the average of 5 different measurements and where all five measurements at at least 5 mm apart from each other.

Step (a) comprises applying a forming means onto the fibres, wherein the forming means orders the composition into a pattern. In at least one embodment, the forming means is pressed against the fibres for a period of time being from 1 second to 55 seconds, or from 1 seconds to 20 seconds, or from 2 seconds to 10 seconds.

In at least one embodment, the forming means is pressed against the fibres at a pressure of from 3 psi to 7 psi. Pounds per square inch = psi.

### Forming means

Step (a) of the first aspect relates to providing fibres coated with a composition and applying a forming means onto the fibres. The forming means orders the composition into a pattern.

In at least one embodiment, the forming means comprises two faces: an upper face and a lower face. In at least one embodiment, the forming means is substantially flat. In at least one embodiment, the lower face is contacted with the coated fibres. In at least one embodiment, the lower face is embossed with a pattern. In at least one embodiment, the forming means is a film and wherein the film comprises an embossed pattern on one side. In at least one embodiment, the pattern is a nano-structured pattern. In at least one embodiment, the nano-structured pattern diffracts light into dispersed colours that are visible on the fibres, preferably visible to the naked eye on the keratin fibres. In at least one embodiment, the nano-structured surface pattern is selected from the group consisting of sawtooth patterns, spiral patterns, ring patterns, Archimedean patterns, ellipsoidal patterns, patterns comprising hyperbolic rings, patterns comprising parabolic rings, and combinations thereof.

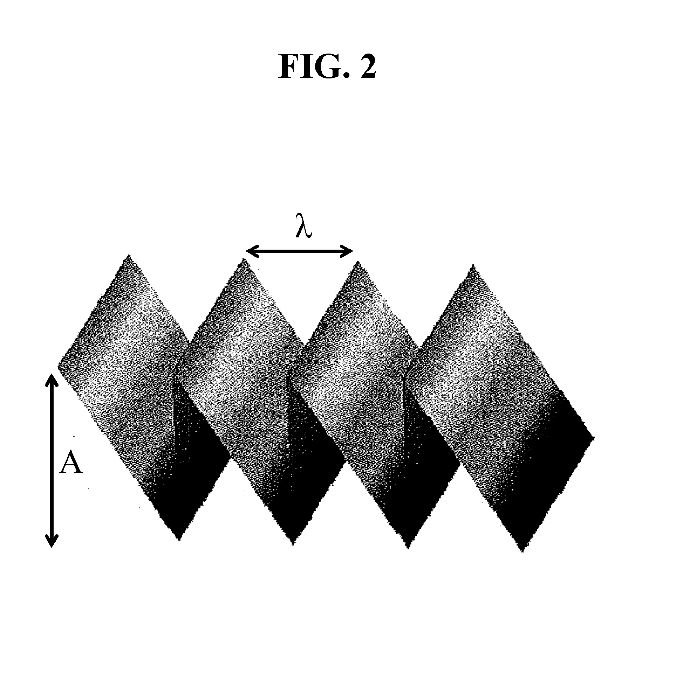

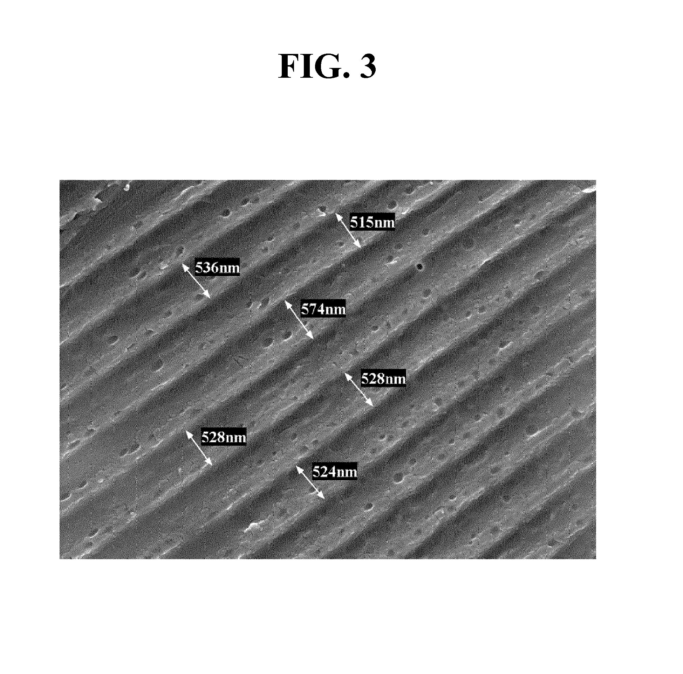

In at least one embodiment, the nano-structured pattern consists of a regular array of peaks and troughs. In at least one embodiment, the nano-structured pattern is a sawtooth pattern. In at least one embodiment, the nano-structured pattern consists of a regular array of peaks and troughs, wherein the distance between peaks is distance λ (lambda), wherein λ is from 200 nm to 700 nm, or from 300 nm to 600 nm, or from 400 nm to 580 nm, or from 500 nm to 550 nm. In at least one embodiment, the nano-structured pattern consists of a regular array of peaks and troughs, wherein the trough depth is distance A, wherein A is from 2 nm to 100 nm, or from 5 nm to 80 nm, or from 10 nm to 50 nm, or from 15 nm to 25 nm. Distances A and λ are depicted in Fig. 2. Distance λ is also depicted in Fig. 3. In at least one embodiment, the average distance λ and the average distance A vary across the nanostructured pattern by max 25%, or max 10%, or max 5%. For example, where the distance λ is measured at five points across the nanostructured pattern and the distance A was found to be 20.5 nm, 21.0 nm, 22.0 nm, 20.0 nm and 21.8 nm, then the average distance A is 20.06 nm. The difference between the average distance A and the furthest away endpoint is 1.06 nm and thus (1.06/21.06)*100 = 5.03%. Therefore, the average distance A varies across the nanostructured pattern by max 5%.

The forming means is a conductor. By "conductor" it is meant that the forming means conducts thermal energy. In at least one embodiment, the forming means has a thermal conductivity of at least 0.1 Watts per meter Kelvin at 23°C, or at least 0.2 Watts per meter Kelvin at 23°C. Thermal conductance is the quantity of heat that passes in unit time through a plate of particular area and thickness when its opposite faces differ in temperature by one Kelvin. Thermal conductivity is measured in Watts per meter Kelvin.

In at least one embodiment, the forming means is permeable to UV radiation. "UV radiation" means electromagnetic radiation in a wavelength range of from 200 nm to 400 nm. UV means ultraviolet. Permeability to UV radiation can be measured using a UV transmission meter, for example the LS108H from LinShang Technology (Shenzhen Linshang Technology Co. Ltd), Guangdong province, China (http://www.lsmeter.com/). In at least one embodiment, the forming means has a UV transmittance of at least 30%, or at least 40%, or at least 50%, or at least 60%, or at least 70%, or at least 80%, or at least 90%, measured using UV light at 365 nm.

In at least one embodiment, the forming means is a film. In at least one embodment, the forming means film has a mean thickness of from 0.1 micron to 2.0 micron, or from 0.3 micron to 1.0 micron. In at least one embodiment, the forming means is a flexible film.

In at least one embodiment, the forming means is made of plastics. In at least one embodiment, the forming means is a film made of a polyolefin resin. Many different blends of components may be used in the polyolefin and components are selected for a variety of properties such as strength and UV permeability. Polyethylene (e.g., Low Density Polyethylene LDPE, Linear Low Density Polyethylene LLDPE, High Density Polyethylene HDPE, Medium Density Polyethylene MDPE, Metallocene Polyethylene mPE, Ethyl Vinyl Acetate EVA and mixtures thereof) and polypropylene, and blends thereof are types of materials that are often used to manufacture films being suitable for the forming means of the present invention. Films suitable as forming means for the present invention may be manufactured using blown film, cast film, and extrusion base processes. The film as forming means, for example, may comprise an 500 nm sandwich consisting of a 200 nm polyethylene layer laminated to a 300 nm polyethylene layer.

Suitable forming means' are available from Breit Technologies, 9084 Bond Street, Overland Park, KS 66214, US (http://www.breit-tech.com/). Their Cast and Cure™ film range is suitable as forming means for the present invention. The Cast and Cure™ film range is created by the Cast & Cure™ process, which is performed by laminating a casting template with a wet UV-curable coating or varnish. While these two surfaces are in contact, ultraviolet (UV) light is passed through the template to cure the varnish. Then the template is delaminated from the surface and rewound for future use. No material is transferred from the template onto the substrate. Also no varnish is transferred to the template. This allows for multiple uses of the template. The template is acting as an embossing tool to manipulate the surface of the coating on a submicron scale. Unlike other holographic options, this process creates an illusion, with no actual transfer of substance.

A forming means may also be prepared using, for example, focused ion beam (FIB), photonic lithography, e-beam lithography, tool machining, ruling engines, diamond turning devices, and any other method or device that can produce nanometer scale features.

### Electromagnetic radiation

The method comprises (b) raising the temperature of the forming means to a temperature of from 40°C to 150°C for up to 15 minutes. The temperature of the forming means can be measured using a temperature probe. In at least one embodment, the step (b) causes the monomer to polymerise into a polymer.

In at least one embodment, the step (b) is carried out by raising the temperature of the forming means to a temperature of from 40°C to 60°C for up to 15 minutes. In at least one embodiment, the heating is carried out for a period of time being from 1 second to 10 seconds, or from 2 seconds to 8 seconds, or from 3 seconds to 5 seconds. It is advantageous to keep the irradiation time to a minimum to avoid heat damage to the fibres. Furthermore and for the same reasons, it is advantageous to keep the temperature as low as possible.

In at least one embodment, in the step (b) method comprises irradiating the forming means with electromagnetic radiation having a wavelength of from 300 nm to 750 nm. In at least one embodiment, the electromagnetic radiation has a wavelength of from 380 nm to 600 nm. In at least one embodment, the electromagnetic radiation having a wavelength of from 200 nm to 400 nm. In at least one embodment, the electromagnetic radiation having a wavelength of from 380 nm to 480 nm.

In at least one embodiment, the irradiation is carried out for a period of time being from 1 second to 10 seconds, or from 2 seconds to 8 seconds, or from 3 seconds to 5 seconds. It is advantageous to keep the irradiation time to a minimum to avoid UV damage to the fibres.

### Polymer

In at least one embodment, the step (b) causes the monomer to polymerise into a polymer. In at least one embodment, the polymer has a glass transition temperature greater than 40°C, or greater than 50°C, or greater than 60°C.

In at least one embodment, the polymer formed is a crosslinked copolymer or a non-crosslinked copolymer. In at least one embodment, the polymer formed is a crosslinked terpolymer or non-crosslinked terpolymer.

In at least one embodment, the composition solifies to form a film having a mean thickness of from 2 nm to 1000 nm, or from 10 nm to 200 nm, or from 20 nm to 180 nm, or from 30 nm to 100 nm. The mean thickness can be measured with microscopy (SEM). The mean thickness is the average of five different measurements and where all five measurements at at least 5 mm apart from each other. Where the film comprises an array of peaks and troughs, then thickness is measured up to the halfway point of distance A. In other words, the thickness includes half the height of each peak/depth of each trough.

The mean thickness of the film resultant from the coating of the composition on the fibres is important in the context of hair fibres because the typical diameter of a hair fibre is 50 micron to 100 micron. Consequently, it is desirable that the film resultant from the coating of the composition on the fibres is not too thick because having a film with an mean thickness being thicker than diameter of the fibre has disadvantages, particularly in terms of its ability to remain on the fibre (durability) over time as well as altering the mechanical properties of the fibre.

The properties of the fibres resultant from the method of the present invention depends on the properties of the polymer coating resultant from the polymerisation of the monomer(s) in the composition as well as the thickness of the coating that results on the fibres (after solidification and polymerisation).

In the context of hair (keratin) fibres, thick films of very rigid coatings can result in negative hair feel which is not desired by the consumer. Furthermore, highly crosslinked polymer films tend to be very rigid and stiff. Indeed, it is desired by the consumer that the colour effects provided by the present invention do not overly detract from the natural and healthy properties of their hair. On the other hand, some consumers would prefer it if the colour effects are in addition to improved hair fibre mechanical properties, for example where the hair fibre is prone to breakage, which can occur if the hair has been overly processed, such as from regularly bleaching and/or perming.

The balance of monomer chemistries used in the present invention can be adjusted. Copolymers with lower levels of a crosslinking (polyfunctional) monomer can have lower glass transition temperatures and may result in polymers that are less rigid and more flexible.

Copolymers of cross-linking monomers with non-crosslinking monomers where the non-crosslinking monomers have low glass transition temperatures can result in more flexible films.

Films with little to no contribution to the mechanical strength of the hairs can be made with thin films of highly crosslinked films and also with thicker films of lightly cross-linked films and especially with copolymers incorporating significant amounts of low-glass transition temperature, non-crosslinking monomers.

### Visual effects

The method is for providing visual effects on fibres. In at least one embodiment, the visual effects are coloured. The forming means orders the composition into a pattern. In at least one embodiment, the pattern is a nano-structured pattern. In at least one embodiment, the pattern is a nano-structured pattern that is complementary to the pattern on the surface of the forming means. In at least one embodiment, the nano-structured pattern diffracts light into dispersed colours that are visible on the fibres, preferably visible to the naked eye on the keratin fibres.

In at least one embodiment, the nano-structured surface pattern is selected from the group consisting of sawtooth patterns, spiral patterns, ring patterns, Archimedean patterns, ellipsoidal patterns, patterns comprising hyperbolic rings, patterns comprising parabolic rings, and combinations thereof.

An analogous system is disclosed in WO2014/160904A1, which published on 2nd October 2014. In particular, Figs 1 to 4e are incorporated herein by reference. Said figures disclose various visual effects applicable for the present invention and they are summarised in the text of WO2014/160904A1, particularly page 3 in the "Brief Description of the Drawings", which is incorporated herein by reference.

In at least one embodiment, the method provides a temporary colouration effect. The colour effect may be shampooed out of the hair. In at least one embodiment, the method provides a permanent or semi-permanent colouration effect. In at least one embodiment, the colouration effect may be removed by using heat.

In at least one embodiment, the method is used to impart highlighting effects on the hair by treating individual hair strands. In at least one embodiment, the method is applied to other hair-like materials that may be secured to the hair or scalp as a hair extension or by any other means suitable for giving the desired nano-structured effects.

### Exemplary embodiments of the first aspect

At least one embodiment relates to a method for providing visual effects on keratin fibres, wherein the method comprises:
(a) applying a composition onto keratin fibres; and then subsequently applying a forming means onto the keratin fibres, wherein the forming means orders the composition into a pattern, and wherein the forming means is a thermal conductor and wherein the forming means is a film embossed with a nano-structured surface pattern;
(b) raising the temperature of the forming means to a temperature of from 40°C to 60°C for up to 15 minutes; and then
(c) removing the forming means from the fibres;
wherein the composition comprises a mixture of different monomers and a cosmetically acceptable carrier;
wherein the monomer(s) are capable of forming a polymer after exposure to heat;
and wherein the monomer(s) are at least two monomers selected from the group consisting of acrylates monomers, methacrylate monomers, acrylamide monomers, methacrylamide monomers, styrene monomers, vinylpyrrolidone monomers;
and wherein the monomer(s) have a molecular weight in the range from 100 g/mol to 500 g/mol;
and wherein the monomer(s) have a functionality of between 1 and 10;
and wherein the composition has a kinematic viscosity of from 0.5 cSt to 1500 cSt, measured at 23°C;
and wherein the composition is substantially free of polymer.

### 2^{nd} Aspect = Kit

The second aspect relates to a kit for providing visual effects on hair, wherein the kit comprises:
i. A composition comprising a monomer(s) and optionally a cosmetically acceptable carrier; wherein the monomer(s) are capable of forming a polymer after exposure to heat; and wherein the monomer(s) are selected from the group consisting of acrylates monomers, methacrylate monomers, acrylamide monomers, methacrylamide monomers, styrene monomers, vinyl pyrolidinone monomers, vinylpyrrolidone monomers, and mixtures thereof; and wherein the monomer(s) have a molecular weight in the range from 100 g/mol to 5000 g/mol; and wherein the monomer(s) have a functionality of between 1 and 100; and wherein the composition has a kinematic viscosity of from 0.5 cSt to 1500 cSt, measured at 23°C;
ii. A device for applying the composition onto hair;
iii. A forming means;
iv. A source of heat.

In at least one embodiment, kit device for applying the composition onto hair, wherein the device for applying the composition onto hair is selected from the group consisting of: sponges, brushes, combs.

In at least one embodiment, the forming means is a film. Such forming means is described above.

All details vis-à-vis the first aspect are compatible and combinable with the second aspect.

In at least one embodiment, the source of heat is a pair of conventional hair straightening irons.

In at least one embodiment, the source of electromagnetic radiation is a pair of hair straightening irons fitted with a source of electromagnetic radiation. In at least one embodiment, the hair straightening irons are fitted with light emitting diodes. Suitable hair straightening irons are disclosed in US20040206368A1. Alternative hair straightening irons are disclosed in US non-provisional patent application having the serial number 14/577,135, entitled "APPLIANCE FOR SHAPING FIBROUS MATERIAL" and claiming priority from US provisional patent application 61/918,159 filed on 19th December 2013. US non-provisional patent application having the serial number 14/577,135 is incorporated herein by reference.

### 3rd Aspect = Use

A third aspect relates to the use of the kit according to the 2^{nd} aspect for providing visual effects on fibres, such as keratin fibres. All details vis-à-vis the first and second aspects are compatible and combinable with the third aspect.

### EXAMPLES

The following examples further describe and demonstrate embodiments within the scope of the present invention. The examples are given solely for the purpose of illustration, and are not to be construed as limitations of the present invention since many variations thereof are possible without departing from its scope.

### Composition

| Ingredient | Ex.1 | Ex.2 | Ex.3 | Ex.4 | Ex.5 | Ex.6 | Ex.7 | Ex.8 |
|---|---|---|---|---|---|---|---|---|
| trimethylo lpropane triacrylate | 99 | - | 34 | - | - | 49 | - | - |
| pentaerythritol triacrylate | - | 20 | - | - | - | - | 39 | - |
| ethylene glycol dimethacrylate | - | 20 | - | - | - | - | - | - |
| polyethylene glycol dimethacrylate | - | - | - | - | - | - | 60 | - |
| 1,3 butylene glycol diacrylate | - | 19 | - | - | - | - | - | - |
| 1,6 hexanediol diacrylate | - | - | 20 | - | - | - | - | - |
| tripropylene glycol diacrylate | - | - | - | - | - 5 | - | - | 60 |
| propoxylated neopentyl glycol diacrylate | - | 20 | - | - | - | - | - | - |
| pentaerythritol tetra-acrylate | - | 20 | - | 49 | - | - | - | - |
| dipentaerythritol pentaacrylate | - | - | 20 | - | - | - | - | 39 |
| butyl acrylate | - | - | 25 | - | - | - | - | - |
| 2-ethylhexyl methacrylate | - | - | - | 50 | 94 | - | - | - |
| butyl methacrylate | - | - | - | - | - | 50 | - | - |
| Water | QSP | QSP | QSP | QSP | QSP | QSP | QSP | QSP |
| Total | 100 | 100 | 100 | 100 | 100 | 100 | 100 | 100 |

Compositions are prepared as liquids using a cosmetically acceptable carrier where necessary to ensure that the composition is a liquid and that the viscosity is in an appropriate range for the chosen application technique. The composition is applied onto human hair by spraying. The forming means is, for example, CAST AND CURE LS 08 CONCENTRIC CIRCLES film from Breit Technologies. The forming means is applied to the coated human hair, which has been laid on a flat surface. The forming means is lightly but evenly pressed into the hair and heated to a temperature of 60°C for 5 seconds. The forming means is removed from the hair and visual effects observed.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for providing visual effects on fibres, wherein the method comprises:
(a) providing fibres coated with a composition and applying a forming means onto the fibres, wherein the forming means orders the composition into a pattern, and wherein the forming means is a conductor; and then
(b) raising the temperature of the forming means to a temperature of from 40°C to 150°C for up to 15 minutes; and then
(c) removing the forming means from the fibres;
wherein the composition comprises monomer(s) and optionally a cosmetically acceptable carrier;
wherein the monomer(s) are capable of forming a polymer after exposure to heat;
and wherein the monomer(s) are selected from the group consisting of acrylates monomers, methacrylate monomers, acrylamide monomers, methacrylamide monomers, styrene monomers, vinyl pyrolidinone monomers, vinylpyrrolidone monomers, and mixtures thereof;
and wherein the monomer(s) have a molecular weight in the range from 100 g/mol to 5000 g/mol;
and wherein the monomer(s) have a functionality of between 1 and 100;
and wherein the composition has a kinematic viscosity of from 0.5 cSt to 1500 cSt, measured at 23°C.

2. The method according to claim 1, wherein step (a) is carried out by:
• Firstly, coating the fibres with the composition, preferably by spraying the composition onto the fibres, or by coating the fibres with a device such as a sponge, comb, or brush; and then
• Secondly, applying a forming means onto the fibres, wherein the forming means orders the composition into a pattern, and wherein the forming means is a thermal conductor.

3. The method according to any of the preceding claims, wherein in step (b) is carried out by raising the temperature of the forming means to a temperature of from 40°C to 60°C for up to 15 minutes.

4. The method according to any of the preceding claims, wherein the monomer(s) are selected from the group consisting of mono-functional acrylic monomers, bi- or poly-functional vinyl monomers, hydrophilic monomers, non-ionic hydrophobic monomers, monomers having at least one carboxylic function, cationic monomers, and combinations thereof.

5. The method according to any of the preceding claims, wherein the monomer(s) are selected from bi- or poly-functional vinyl monomers; preferably wherein the bi- or poly-functional vinyl monomer is selected from the group consisting of allyl methacrylate, triethylene glycol dimethacrylate, ethylene glycol dimethacrylate, diethylene glycol dimethacrylate, aliphatic or aromatic urethane diacrylates, difunctional urethane acrylates, ethoxylated aliphatic difunctional urethane methacrylates, aliphatic or aromatic urethane dimethacrylates, epoxy acrylates, epoxymethacrylates, tetraethylene glycol dimethacrylate, polyethylene glycol dimethacrylate, 1,3 butylene glycol diacrylate, 1,4-butanediol dimethacrylate, 1,4-butaneidiol diacrylate, diethylene glycol diacrylate, 1,6 hexanediol diacrylate, 1,6 hexanediol dimethacrylate, neopentyl glycol diacrylate, polyethylene glycol diacrylate, tetraethylene glycol diacrylate, triethylene glycol diacrylate, 1,3 butylene glycol dimethacrylate, tripropylene glycol diacrylate, ethoxylated bisphenol diacrylate, ethoxylated bisphenol dimethylacrylate, dipropylene glycol diacrylate, alkoxylated hexanediol diacrylate, alkoxylated cyclohexane dimethanol diacrylate, propoxylated neopentyl glycol diacrylate, trimethylolpropane trimethacrylate, trimethylolpropane triacrylate, pentaerythritol triacrylate, ethoxylated trimethylolpropane triacrylate, propoxylated trimethylolpropane triacrylate, propoxylated glyceryl triacrylate, ditrimethylolpropane tetraacrylate, dipentaerythritol pentaacrylate, ethoxylated pentaerythritol tetraacrylate, and mixtures thereof.

6. The method according to any of the preceding claims, wherein the composition comprises a polymerisation inhibitor.

7. The method according to any of the preceding claims, wherein the cosmetically acceptable carrier is selected from the group consisting of water and aqueous-alcoholic solutions.

8. The method according to any of the preceding claims, wherein the forming means is made of plastics.

9. The method according to any of the preceding claims, wherein the forming means is a film and wherein the film comprises an embossed pattern on one side.

10. The method according to any of the preceding claims, wherein step (b) causes the monomer to polymerise into a polymer.

11. The method according to claim 10, wherein the polymer has a glass transition temperature greater than 40°C, preferably greater than 60°C.

12. The method according to any of the preceding claims, wherein the composition comprises a mixture of monomers.

13. The method according to any of the preceding claims, wherein the composition further comprises an organic photoinitiator.

14. The method according to any of the preceding claims, wherein the composition is substantially free of an organometallic compound.

15. A kit for providing visual effects on fibres, wherein the kit comprises:
i. A composition comprising monomer(s) and optionally a cosmetically acceptable carrier; wherein the monomer(s) are capable of forming a polymer after exposure to heat; and wherein the monomer(s) are selected from the group consisting of acrylates monomers, methacrylate monomers, acrylamide monomers, methacrylamide monomers, styrene monomers, vinyl pyrolidinone monomers, vinylpyrrolidone monomers, and mixtures thereof; and wherein the monomer(s) have a molecular weight in the range from 100 g/mol to 5000 g/mol; and wherein the monomer(s) have a functionality of between 1 and 100; and wherein the composition has a kinematic viscosity of from 0.5 cSt to 1500 cSt, measured at 23°C;
ii. A device for applying the composition onto fibres;
iii. A forming means;
iv. A source of heat.
